# EUROPEAN PATENT SPECIFICATION

(11) **EP 0 334 674 B1**
(45) Date of publication and mention of the grant of the patent: **01.06.1994**
(21) Application number: 89302972.8
(22) Date of filing: 23.03.1989
(51) Int. Cl.: A61K 31/42, A61K 31/445, A61K 31/495, A61K 31/535, A61K 31/55

(54) **Use of isoxazolinones as cerebro-active drugs**
Verwendung von Isoxazolinonen als cerebro-aktive Medikamente
Utilisation d'isoxazolinones comme médicaments à activité cérébrale

(30) Priority: 24.03.1988 JP 70427/88
(43) Date of publication of application: 27.09.1989
(73) Proprietor: Sankyo Company Limited, Tokyo (JP)
(72) Inventor: Iwata, Nobuyoshi, Shinagawa-ku Tokyo, 140 (JP); Kobayashi, Kazuo, Shinagawa-ku Tokyo, 140 (JP); Kubo, Yoshiko, Shinagawa-ku Tokyo, 140 (JP); Kozuka, Masao, Shinagawa-ku Tokyo, 140 (JP); Nagano, Mitsuo, Shinagawa-ku Tokyo, 140 (JP)
(74) Representative: Gibson, Christian John Robert

(56) References cited:
- EP-A- 0 250 241
- EP-A- 0 273 744
- WO-A-86/02268
- GB-A- 2 165 237
- US-A- 3 459 862
- US-A- 4 470 993
- PATENT ABSTRACTS OF JAPAN, vol. 5, no. 89 (C-58)[761], 10th June 1981, page 103 C 58; & JP-A 56 34 674 (SANKYO K.K.) 06-04-1981

## Description

The present invention relates to the use for the manufacture of a medicament for treating patients with cerebrovascular disorders of a series of isoxazolinone derivatives of formula (I), as defined hereafter.

Cerebrovascular disorders, including stroke infarction, are among the main causes of death in the world today. Moreover, although the patient may survive in such cases, cognitive impairment, which may be one of the sequelae of the disease, is a substantial social problem at present. For this reason, the development of therapeutic agents for the treatment of such disorders (commonly referred to as "cerebro-active drugs") is required.

The compounds of formula (I) are known from Japanese Patent Application Kokai (i.e. as laid open to public inspection) No. 56-34674, where their anti-inflammatory, analgesic and anti-pyretic activities are disclosed, and from European Patent Publication No. 273 744, where the same compounds were disclosed to have a centrally acting muscle relaxant activity, which makes them of use for the treatment of the myotony or spasrigido hemiplegia which are often observed as the sequelae of cerebrovascular disorders, such as stroke infarction, or as cerebrotraumatic sequelae. It is thought that the active site for the compounds in treating these disorders described in the prior art is the spinal cord, and that the compounds can therefore relieve problems whose site of action is the spinal cord.

Compounds having certain structural similarities to some of the compounds of formula (I) are disclosed in EP 250241 as having use in the protection of brain cells and, in particular for the treatment of dementia.

On the other hand, we have now discovered that these compounds also have a direct effect on the cerebral circulation and/or metabolism, and can be used in the treatment of cerebrovascular disorders. Unlike the case of the prior art, where the site(s) of action is the spinal cord, when the compounds are used in accordance with the present invention, the main site of action is believed to be the hippocampus, and the compounds of the present invention thus help relieve disorders caused by the problems with cerebral circulation.

The cerebro-active drugs of the present invention are those isoxazolin-3-one derivatives of formula (I): 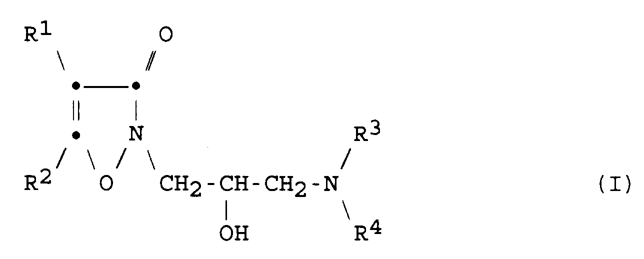
in which:
R¹ represents a hydrogen atom or a halogen atom;
R² represents a C₁ - C₄ alkyl group, a phenyl group, a substituted phenyl group having at least one of substituents (a); or R¹ and R², together with the adjacent carbon atoms form a hydrocarbon ring having 6 or 7 ring carbon atoms;
R³ represents a hydrogen atom or a C₁ - C₄ alkyl group;
R⁴ represents a C₁ - C₄ alkyl group or R³ and R⁴, together with the adjacent nitrogen atom, form a morpholino, 1-piperazinyl, 4-methyl-1-piperazinyl or piperidino group, or form a cycloaliphatic amino group having 5 or 6 ring atoms, said cycloaliphatic amino group being unsubstituted or having at least one of substituents (a), defined below;
substituents (a):
C₁ - C₄ alkyl groups, C₁ - C₄ alkoxy groups, hydroxy groups, halogen atoms, nitro groups, and trifluoromethyl groups;
and pharmacologically acceptable acid addition salts thereof.

The present invention thus provides the use for the manufacture of a medicament for treating cerebrovascular disoders of a compound of formula (I) or a salt thereof, as defined above.

In the compounds of the present invention, where R¹ represents a halogen atom, this is preferably a fluorine, chlorine or bromine atom.

Where R², R³ or R⁴ represents an alkyl group, this may be a straight or branched alkyl group having from 1 to 4 carbon atoms, and examples include the methyl, ethyl, propyl, isopropyl, butyl, isobutyl and t-butyl groups. Where R² represents a phenyl group, this may be unsubstituted or it may have at least one of substituents (a), defined above. Examples of such substituents include:
alkyl groups having from 1 to 4, preferably from 1 to 3, carbon atoms, e.g. the methyl, ethyl, propyl, isopropyl, butyl, isobutyl and t-butyl groups, preferably the methyl, ethyl, propyl and isopropyl groups;
alkoxy groups having from 1 to 4, preferably from 1 to 3, carbon atoms, e.g. the methoxy, ethoxy, propoxy, isopropoxy, butoxy, isobutoxy and t-butoxy groups, preferably the methoxy, ethoxy, propoxy and isopropoxy groups;
halogen atoms, such as the fluorine, chlorine and bromine atoms;
the nitro group; and
the trifluoromethyl group.

Alternatively, R¹ and R², together with the adjacent carbon atoms, may form a 6- or 7-membered hydrocarbon ring, which may be aliphatic or aromatic in character, such as a benzene ring, cyclohexene ring or cycloheptene ring.

Where R³ does not represent a hydrogen atom or an alkyl group and R⁴ does not represent an alkyl group, they may, together with the adjacent nitrogen atom, form a 5- or 6-membered cycloaliphatic amino group such as a morpholino, 1-piperazinyl, 4-methyl-1-piperazinyl, 1-pyrrolidinyl or piperidino group.

The compounds of the present invention are basic and can, therefore, form acid addition salts. The use of such salts, insofar as they are pharmaceutically acceptable, also forms part of the present invention. Examples of pharmaceutically acceptable acid addition salts include:
mineral acid salts, such as the hydrochloride, hydrobromide and sulphate; organic carboxylic acid salts, such as the oxalate, lactate, citrate, tartarate, succinate, maleate and fumarate; and organic sulphonic acid salts, such as the methanesulphonate.

It should be noted that the compounds of formula (I) can exist in the form of optical isomers because of the presence of asymmetric carbon atoms. The present invention envisages the use both of the individual, isolated isomers, as well as mixtures thereof, whether racemic mixtures or otherwise.

Preferred compounds of the present invention include those of formula (I), in which:
R¹ represents a hydrogen atom or a halogen atom;
R² represents a phenyl group or a phenyl group having at least one of substituents (a), defined above; or R¹ and R², together with the adjacent carbon atoms form a benzene ring;
R³ represents a hydrogen atom; and
R⁴ represents a C₁ - C₄ alkyl group or R³ and R⁴, together with the adjacent nitrogen atom, form a cycloaliphatic amino group having 5 or 6 ring atoms, said cycloaliphatic amino group being unsubstituted or having at least one of substituents (a), defined above;
and pharmacologically acceptable acid addition salts thereof.

The more preferred compounds of the present invention include those of formula (I), in which:
R¹ represents a hydrogen, fluorine, chlorine or bromine atom;
R² represents a phenyl group or a phenyl group having at least one of substituents (b), defined below;
R³ and R⁴, together with the adjacent nitrogen atom, form a morpholino, 1-piperazinyl, 4-methyl-1-piperazinyl or piperidino group; and
substituents (b):
C₁ - C₄ alkyl groups, C₁ - C₄ alkoxy groups, hydroxy groups, fluorine atoms, chlorine atoms and bromine atoms;
and pharmacologically acceptable acid addition salts thereof.

The most preferred compounds of the present invention include those of formula (I), in which:
R¹ represents a hydrogen atom or a chlorine atom;
R² represents a phenyl group or a phenyl group having at least one halogen substituent; and
R³ and R⁴, together with the adjacent nitrogen atom, form a morpholino, 1-piperazinyl, 4-methyl-1-piperazinyl or piperidino group;
and pharmacologically acceptable acid addition salts thereof.

Specific examples of compounds which may be used in the present invention include the following:
2-(2-hydroxy-3-morpholinopropyl)-5-phenyl-4-isoxazolin-3-one;
4-chloro-2-(2-hydroxy-3-morpholinopropyl)-5-phenyl-4-isoxazolin-3-one;
2-(2-hydroxy-3-morpholinopropyl)-5-methyl-4-isoxazolin-3-one;
5-(p-chlorophenyl)-2-(2-hydroxy-3-morpholinopropyl)-4-isoxazolin-3-one;
4-chloro-2-(2-hydroxy-3-isopropylaminopropyl)-5-phenyl-4-isoxazolin-3-one;
2-(2-hydroxy-3-isopropylaminopropyl)-5-phenyl-4-isoxazolin-3-one; and
5-(p-chlorophenyl)-2-(2-hydroxy-3-isopropylaminopropyl)-4-isoxazolin-3-one;
2-(2-hydroxy-3-morpholinopropyl)-2,3-dihydro-1,2-benzoisoxasol-3-one;
2-(2-hydroxy-3-morpholinopropyl)-2,3,4,5,6,7-hexa-hydro-1,2-bensoisoxazol-3-one;
and pharmaceutically acceptable acid addition salts thereof, especially the hydrochloride.

Of these, the following are most preferred:

### Compound (1):

2-(2-hydroxy-3-morpholinopropyl)-5-phenyl-4-isoxazolin-3-one and its hydrochloride;

### Compound (2);

4-chloro-2-(2-hydroxy-3-morpholinopropyl)-5-phenyl-4-isoxazolin-3-one and its hydrochloride; and

### Compound (3):

5-(p-chlorophenyl)-2-(2-hydroxy-3-morpholinopropyl)-4-isoxazolin-3-one and its hydrochloride.

The isoxazoline derivatives of formula (I), which are the active ingredients employed in this invention, may be prepared by the method described in the specification of Japanese Patent Application Kokai No. 56-34674.

Specifically, the compounds of the present invention can be prepared by reacting a 2-(3-halo-2-hydroxy-propyl)-4-isoxazolin-3-one of formula (II): 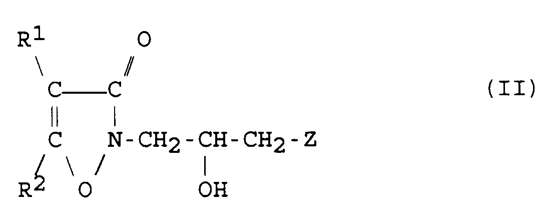
(in which R¹ and R² are as defined above and Z represents a halogen atom, for example a chlorine, bromine or iodine atom) with an amine of formula (III): 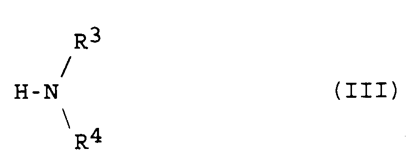
(in which R³ and R⁴ are as defined above).

For example, the compound of formula (II) may be reacted with the amine of formula (III) in a suitable solvent under reflux for several hours, and the product may then be isolated by conventional techniques. Further details can be found in Japanese Patent Application Kokai No. 56-34674.

As shown in the pharmacological activity and toxicity tests described in the following Experiments, the compounds of the present invention have an excellent ability to counter some symptoms elicited by cerebral ischemia, combined with a low toxicity.

### EXPERIMENT 1

### Improvement of ischemia-induced neurological symptoms in the Mongolian Gerbil caused by ligation of the bilateral common carotid arteries for 30 minutes

The test animals employed were female adult (16 to 17 weeks old) Mongolian gerbils. These were used in groups each consisting of 17 animals. The bilateral common carotid arteries were occluded for 30 minutes under anesthesia with pentobarbital (30 mg/kg, intraperitoneally) and halothane (it was introduced to a mixture of 95% oxygen and 5% carbon dioxide at a concentration of 1.5% by volume) and then the occlusion was released to allow the blood to flow. The animal was then placed in a supine position, and we measured (1) the time taken from recommencement of blood flow until convulsions occurred and (2) the survival time. Observations to determine the onset time of convulsions and the survival time were continued for 6 and 7 hours, respectively, after recommencement of blood flow. If no convulsions occurred within 6 hours after the recommencement of blood flow, the onset time of convulsions was assumed to be 360 minutes, and, if the animal did not die within 7 hours, the survival time was recorded as 420 minutes for calculation purposes. The compound to be tested was dissolved or suspended in a 0.5% w/v carboxymethyl cellulose (CMC) aqueous solution and administered intraperitoneally upon recommencement of blood flow in the common carotid arteries. The dose of test compound was 100 mg/kg, and the compound used was Compound No. 2, defined above. On the other hand, a 0.5% CMC solution containing no test compound was administered likewise to a control group. Statistical analysis was carried out using the Mann-Whitney U-test between the control group and the test group.

The results are shown in Table 1. As can be seen from these results, in the group to which the hydrochloride of the above Compound No. 2 was administered at a dose of 100 mg/kg, both onset time of convulsions and survival time were significantly prolonged (p <0.01).

**Table 1**

| Test Compound | Onset time of convulsions (min.) | Survival time (min.) |
|---|---|---|
| Compound No. 2 hydrochloride | 289.4 ± 11.9 | 375.7 ± 10.5 |
| None (Control) | 198.8 ± 19.9 | 300.6 ± 14.3 |

### EXPERIMENT 2

### Improvement in ischemia-induced neurological symptoms in Spontaneously Hypertensive Rats Stroke Prone (SHR SP) caused by ligation of the bilateral common carotid arteries

The test animals employed were male adult SHR SPs (15 weeks old). They were used in groups each consisting of 11 animals. Under halothane anesthesia, both common carotid arteries of each animal were ligated to prepare a brain ischemia model. The halothane anesthesia was stopped immediately after the ligation, and then the time taken to recover the righting reflex, the onset time of convulsions and the survival time were determined. The test solution was prepared in the same manner as described in Experiment 1 and was administered to the animal intraperitoneally 30 minutes before the ligation. A 0.5% CMC solution was administered to the control group in the same manner. The statistical analysis was carried out in a similar manner to that in Experiment 1.

The results are shown in Table 2, from which it can be seen that administration of the hydrochloride of Compound No. 2 at doses of 30 and 100 mg/kg shortened significantly the time taken to recover the righting reflex (p <0.02) and it prolonged significantly the onset time of convulsions (p <0.01) at a dose of 100 mg/kg. In the Table, the times are all reported in minutes.

**Table 2**

| Test Compound | Dose (mg/kg) | Recovery time of righting reflex (min.) | Onset time of convulsions (min.) | Survival time (min.) |
|---|---|---|---|---|
| Compound No. 2 hydrochloride | 30 | 8.0 ± 1.7 | 99.8 ± 27.8 | 232.1 ± 35.8 |
| | 100 | 7.6 ± 1.7 | 103.5 ± 16.2 | 233.0 ± 28.7 |
| Control | - | 15.5 ± 2.1 | 50.5 ± 6.6 | 226.7 ± 26.7 |

### EXPERIMENT 3

### Acute toxicity

The hydrochloride of Compound No. 2 was dissolved in a 0.5% CMC solution, and the resulting solution was administered orally at a dose of 500 mg/kg to five mice. During the period of observation (5 days), all mice survived.

As can be seen from the results described above, the compounds of the present invention have a so-called cerebroprotective effect, which improved ischemia-induced neurological symptoms caused by ligation of the bilateral common carotid arteries, without inducing sleep and with an extremely low toxicity. It has been found that the compounds of the present invention are absorbed very well by the oral route, and, furthermore, since the hydrochloride is soluble in water, it is clinically possible for it to be administered intravenously or orally, as required. Although it had already been found that the compounds of the present invention have a centrally acting muscle relaxant activity (as shown in European Patent Publication No. 273 744), it could not have been predicted from this that they would also be useful as cerebro-active drugs for the treatment of the acute and chronic phases of cerebral stroke infarction or for the postoperative treatment of patients with a cerebral tumor, a head injury, or the like.

The compounds of the present invention may therefore be used in the treatment of such disorders, and, for this purpose, may be formulated as conventional pharmaceutical preparations, as is well known in the art. Thus, the compounds may be administered orally, e.g. in the form of tablets, capsules, granules, powders, syrups, or other such well known forms, or parenterally, e.g. by injections, suppositories, etc.

These pharmaceutical preparations can be prepared by conventional means and may contain known adjuvants of a type commonly used in this field, for example vehicles, binders, disintegrators, lubricants, correctives, etc. depending upon the intended use and form of the preparation. The dose will depend upon the condition, age, and body weight of the patient as well as upon the nature and severity of the disorder to be treated, but in the case of oral administration to an adult human patient, we would normally suggest a total daily dose of from 5 mg to 50 mg, which may be administered in a single dose or in divided doses, e.g. from one to three times a day.

The following Example illustrates the preparation of a pharmaceutical preparation in accordance with the present invention.

The following powders were mixed:

| | |
|---|---|
| 4-Chloro-2-(2-hydroxy-3-morpholinopropyl)-5-phenyl-4-isoxazolin-3-one hydrochloride (Hydrochloride of Compound No. 2) | 25.0 mg |
| Lactose | 153.6 mg |
| Corn starch | 100.0 mg |
| Magnesium stearate | 1.4 mg |
| Total : | 280.0 mg |

and the resulting mixture was passed through a 60 mesh (Tyler standard mesh) sieve. 280 mg of the resulting powdered mixture was packed into a No. 3 gelatin capsule.

## Claims

1. The use for the manufacture of a medicament for the treatment of cerebrovascular disorders of a cerebro-active drug, wherein the cerebro-active drug is an isoxazolin-3-one derivative of formula (I): 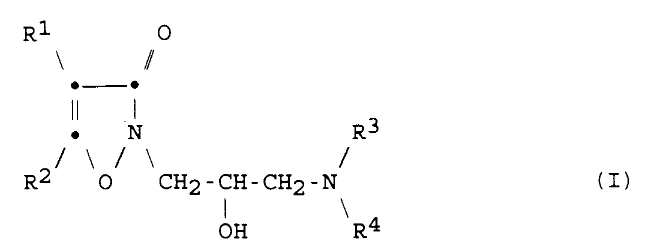 in which:
R¹ represents a hydrogen atom or a halogen atom;
R² represents a C₁ - C₄ alkyl group, a phenyl group, a substituted phenyl group having at least one of substituents (a), or R¹ and R², together with the adjacent carbon atoms form a hydrocarbon ring having 6 or 7 ring carbon atoms;
R³ represents a hydrogen atom or a C₁ - C₄ alkyl group;
R⁴ represents a C₁ - C₄ alkyl group or R³ and R⁴, together with the adjacent nitrogen atom, form a morpholino, 1-piperazinyl, 4-methyl-1-piperazinyl or piperidino group, or form a cycloaliphatic amino group having 5 or 6 ring atoms, said cycloaliphatic amino group being unsubstituted or having at least one of substituents (a), defined below;
substituents (a):
C₁ - C₄ alkyl groups, C₁ - C₄ alkoxy groups, hydroxy groups, halogen atoms, nitro groups, and trifluoromethyl groups;
or a pharmacologically acceptable acid addition salt thereof.

2. The use according to Claim 1, in which R² represents a phenyl group or a substituted phenyl group having at least one of substituents (a), defined in Claim 1; or R¹ and R², together with the adjacent carbon atoms form a benzene ring.

3. The use according to any one of Claims 1 to 2, in which R³ represents a hydrogen atom.

4. The use according to any one of Claims 1 to 3, in which R⁴ represents a C₁ - C₄ alkyl group or R³ and R⁴, together with the adjacent nitrogen atom, form a cycloaliphatic amino group having 5 or 6 ring atoms, said cycloaliphatic amino group being unsubstituted or having at least one of substituents (a), defined in Claim 1.

5. The use according to Claim 1, in which:
R¹ represents a hydrogen atom or a halogen atom;
R² represents a phenyl group or a substituted phenyl group having at least one of substituents (a), defined in Claim 1; or R¹ and R², together with the adjacent carbon atoms form a benzene ring;
R³ represents a hydrogen atom; and
R⁴ represents a C₁ - C₄ alkyl group or R³ and R⁴, together with the adjacent nitrogen atom, form a cycloaliphatic amino group having 5 or 6 ring atoms, said cycloaliphatic amino group being unsubstituted or having at least one of substituents (a), defined in Claim 1.

6. The use according to Claim 1, in which:
R¹ represents a hydrogen, fluorine, chlorine or bromine atom;
R² represents a phenyl group or a substituted phenyl group having at least one of substituents (b), defined below;
R³ and R⁴, together with the adjacent nitrogen atom, form a morpholino, 1-piperazinyl, 4-methyl-1-piperazinyl or piperidino group;
substituents (b):
C₁ - C₄ alkyl groups, C₁ - C₄ alkoxy groups, hydroxy groups, fluorine atoms, chlorine atoms and bromine atoms.

7. The use according to Claim 1, in which:
R¹ represents a hydrogen atom or a chlorine atom;
R² represents a phenyl group or a substituted phenyl group having at least one halogen substituent; and
R³ and R⁴, together with the adjacent nitrogen atom, form a morpholino, 1-piperazinyl, 4-methyl-1-piperazinyl or piperidino group.

8. The use according to Claim 1, in which said isoxazolin-3-one derivative is 2-(2-hydroxy-3-morpholinopropyl)-5-phenyl-4-isoxazolin-3-one or a pharmacologically acceptable acid addition salt thereof.

9. The use according to Claim 1, in which said isoxazolin-3-one derivative is 2-(2-hydroxy-3-morpholinopropyl)-5-phenyl-4-isoxazolin-3-one or its hydrochloride.

10. The use according to Claim 1, in which said isoxazolin-3-one derivative is 4-chloro-2-(2-hydroxy-3-morpholinopropyl)-5-phenyl-4-isoxazolin-3-one or a pharmacologically acceptable acid addition salt thereof.

11. The use according to Claim 1, in which said isoxazolin-3-one derivative is 4-chloro-2-(2-hydroxy-3-morpholinopropyl)-5-phenyl-4-isoxazolin-3-one or its hydrochloride.

12. The use according to Claim 1, in which said isoxazolin-3-one derivative is 5-(p-chlorophenyl)-2-(2-hydroxy-3-morpholinopropyl)-4-isoxazolin-3-one or a pharmacologically acceptable acid addition salt thereof.

13. The use according to Claim 1, in which said isoxazolin-3-one derivative is 5-(p-chlorophenyl)-2-(2-hydroxy-3-morpholinopropyl)-4-isoxazolin-3-one or its hydrochloride.

14. The use according to any one of the preceding Claims, wherein said active compound is formulated for administration by the oral route or by the parenteral route.

15. The use according to any one of the preceding Claims, wherein said active compound is formulated as tablets, capsules, granules, powders, syrups, injections or suppositories.

16. The use according to any one of the preceding Claims, wherein said active compound is formulated for administration at a dose of 5 mg to 50 mg orally to adult humans, 1 to 3 times a day.

## Patentansprüche

1. Verwendung eines cerebro-aktiven Arzneimittels zur Herstellung eines Arzneimittels zur Behandlung von cerebrovaskulären Erkrankungen, wobei das cerebro-aktive Arzneimittel ein Isoxazolin-3-on-Derivat der Formel (I): 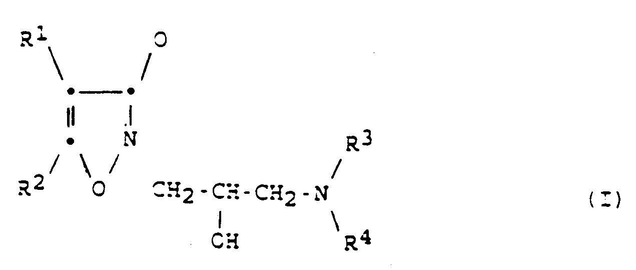 worin
R¹ ein Wasserstoffatom oder ein Halogenatom bedeutet,
R² eine C₁ - C₄-Alkylgruppe, eine Phenylgruppe, eine substituierte Phenylgruppe, die mindestens einen der Substituenten (a) aufweist, bedeutet, oder R¹ und R² zusammen mit den benachbarten Kohlenstoffatomen einen Kohlenwasserstoffring mit 6 oder 7 Ringkohlenstoffatomen bilden,
R³ ein Wasserstoffatom oder eine C₁ - C₄-Alkylgruppe bedeutet,
R⁴ eine C₁ - C₄-Alkylgruppe bedeutet oder R³ und R⁴ gemeinsam mit dem benachbarten Stickstoffatom eine Morpholino-, 1-Piperazinyl-, 4-Methyl-1-piperazinyl oder eine Piperidinogruppe bilden oder eine cycloaliphatische Aminogruppe mit 5 oder 6 Ringatomen bilden, wobei die cycloaliphatische Aminogruppe unsubstituiert ist oder mindestens einen der nachstehend definierten Substituenten (a) aufweist.
Substituenten (a):
C₁ - C₄-Alkylgruppen, C₁ - C₄-Alkoxygruppen, Hydroxygruppen, Halogenatome, Nitrogruppen und Trifluormethylgruppen;
oder ein pharmazeutisch verträgliches Säureadditionsalz davon ist.

2. Verwendung nach Anspruch 1, wobei R² eine Phenylgruppe oder eine substituierte Phenylgruppe, die mindestens einen der in Anspruch 1 definierten Substituenten (a) aufweist, bedeutet oder R¹ und R² zusammen mit den benachbarten Kohlenstoffatomen einen Benzolring bilden.

3. Verwendung nach einem der Ansprüche 1 bis 2, wobei R³ ein Wasserstoffatom bedeutet.

4. Verwendung nach einem der Ansprüche 1 bis 3, wobei R⁴ eine C₁ - C₄-Alkylgruppe bedeutet oder R³ und R⁴ zusammen mit dem benachbarten Stickstoffatom eine cycloaliphatische Aminogruppe mit 5 oder 6 Ringatomen bilden, wobei die cycloaliphatische Aminogruppe unsubstituiert ist oder mindestens einen der in Anspruch 1 definierten Substituenten (a) aufweist.

5. Verwendung nach Anspruch 1, wobei:
R¹ ein Wasserstoffatom oder ein Halogenatom bedeutet,
R² eine Phenylgruppe oder eine substituierte Phenylgruppe, die mindestens einen der in Anspruch 1 definierten Substituenten (a) aufweist, bedeutet, oder R¹ und R² zusammen mit den benachbarten Kohlenstoffatomen einen Benzolring bilden,
R³ ein Wasserstoffatom bedeutet und
R⁴ eine C₁ - C₄-Alkylgruppe bedeutet oder R³ und R⁴ zusammen mit dem benachbarten Stickstoffatom eine cycloaliphatische Aminogruppe mit 5 oder 6 Ringatomen bilden, wobei die cycloaliphatische Aminogruppe unsubstituiert ist oder mindestens einen der in Anspruch 1 definierten Substituenten (a) aufweist.

6. Verwendung nach Anspruch 1, wobei
R¹ ein Wasserstoff-, Fluor-, Chlor- oder Bromatom bedeutet,
R² eine Phenylgruppe oder eine substituierte Phenylgruppe, die mindestens einen der nachstehend definierten Substituenten (b) aufweist, bedeutet,
R³ und R⁴ zusammen mit dem benachbarten Stickstoffatom eine Morpholino-, 1-Piperazinyl-, 4-Methyl-1-piperazinyl- oder Piperidinogruppe bilden,
Substituenten (b):
C₁ - C₄-Alkylgruppen, C₁ - C₄-Alkoxygruppen, Hydroxygruppen, Fluoratome, Chloratome und Bromatome.

7. Verwendung nach Anspruch 1, wobei
R¹ ein Wasserstoffatom oder ein Chloratom bedeutet,
R² eine Phenylgruppe oder eine substituierte Phenylgruppe bedeutet, die mindestens einen Halogensubstituenten aufweist, und
R³ und R⁴ zusammen mit dem benachbarten Stickstoffatom eine Morpholino-, 1-Piperazinyl-, 4-Methyl-1-piperazinyl- oder Piperidinogruppe bilden.

8. Verwendung nach Anspruch 1, wobei das Isoxazolin-3-on-Derivat 2-(2-Hydroxy-3-morpholinopropyl)-5-phenyl-4-isoxazolin-3-on oder ein pharmazeutisch verträgliches Säureadditionsalz davon ist.

9. Verwendung nach Anspruch 1, wobei das Isoxazolin-3-on-Derivat 2-(2-Hydroxy-3-morpholinopropyl)-5-phenyl-4-isoxazolin-3-on oder dessen Hydrochlorid ist.

10. Verwendung nach Anspruch 1, wobei das Isoxazolin-3-on-Derivat 4-Chlor-2-(2-hydroxy-3-morpholinopropyl)-5-phenyl-4-isoxazolin-3-on oder ein pharmazeutisch verträgliches Säureadditionsalz davon ist.

11. Verwendung nach Anspruch 1, wobei das Isoxazolin-3-on-Derivat 4-Chlor-2-(2-hydroxy-3-morpholinopropyl)-5-phenyl-4-isoxazolin-3-on oder dessen Hydrochlorid ist.

12. Verwendung nach Anspruch 1, wobei das Isoxazolin-3-on-Derivat 5-(p-Chlorphenyl)-2-(2-hydroxy-3-morpholinopropyl-4-isoxazolin-3-on oder ein pharmazeutisch verträgliches Säureadditionsalz davon ist.

13. Verwendung nach Anspruch 1, wobei das Isoxazolin-3-on-Derivat 5-(p-Chlorphenyl)-2-(2-hydroxy-3-morpholinopropyl)-4-isoxazolin-3-on oder dessen Hydrochlorid ist.

14. Verwendung nach einem der vorhergehenden Ansprüche, wobei die aktive Verbindung für die Verabreichung auf oralem Weg oder auf parenteralem Weg formuliert ist.

15. Verwendung nach einem der vorhergehenden Ansprüche, wobei die aktive Verbindung in Form von Tabletten, Kapseln, Granulat, Pulver, Sirup, Injektionsflussigkeit oder Suppositorien ausgebildet ist.

16. Verwendung nach einem der vorhergehenden Ansprüche, wobei die aktive Verbindung so formuliert ist, daß sie 1 bis 3 mal pro Tag in einer Dosis von 5 mg bis 50 mg oral an Erwachsene verabreicht wird.

## Revendications

1. Utilisation pour la production d'un médicament pour le traitement des troubles cérébro-vasculaires d'un médicament à activité cérébrale, dans laquelle le médicament à activité cérébrale est un dérivé d'isoxazoline-3-one de formule (I) : 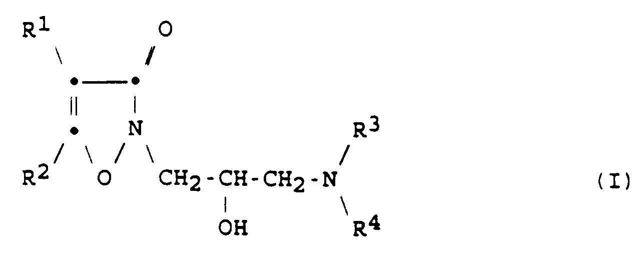 dans laquelle :
R¹ représente un atome d'hydrogène ou un atome d'halogène ;
R² représente un groupe alkyle en C₁-C₄, un groupe phényle, un groupe phényle substitué ayant au moins un substituant (a), ou R¹ et R², avec les atomes de carbone adjacents, forment un cycle hydrocarboné ayant 6 ou 7 atomes de carbone formant le cycle ;
R³ représente un atome d'hydrogène ou un groupe alkyle en C₁-C₄ ;
R⁴ représente un groupe alkyle en C₁-C₄ ou R³ et R⁴, avec l'atome d'azote adjacent, forment un groupe morpholino, 1-pipérazinyle, 4-méthyl-1-pipérazinyle ou pipéridino ou forment un groupe amino cycloaliphatique ayant 5 ou 6 atomes formant le cycle, ledit groupe amino cycloaliphatique étant non substitué ou ayant au moins un des substituants (a) définis ci-après ;
substituants (a) :
groupes alkyle en C₁-C₄, groupes alcoxy en C₁-C₄, groupes hydroxy, atomes d'halogène, groupes nitro et groupes trifluorométhyle ;
ou un sel d'addition d'acide pharmacologiquement acceptable de celui-ci.

2. Utilisation selon la revendication 1, où R² représente un groupe phényle ou un groupe phényle substitué ayant au moins un des substituants (a) définis dans la revendication 1 ; ou R¹ et R², avec les atomes de carbone adjacents, forment un cycle benzène.

3. Utilisation selon l'une des revendications 1 ou 2, où R³ représente un atome d'hydrogène.

4. Utilisation selon l'une quelconque des revendications 1 à 3, où R⁴ représente un groupe alkyle en C₁-C₄ ou R³ et R⁴, avec l'atome d'azote adjacent, forment un groupe amino cycloaliphatique ayant 5 ou 6 atomes formant le cycle, ledit groupe amino cycloaliphatique étant non substitué ou ayant au moins un des substituants (a) définis dans la revendication 1.

5. Utilisation selon la revendication 1, où :
R¹ représente un atome d'hydrogène ou un atome d'halogène ;
R² représente un groupe phényle ou un groupe phényle substitué ayant au moins un des substituants (a) définis dans la revendication 1 ; ou R¹ et R², avec les atomes de carbone adjacents, forment un cycle benzène ;
R³ représente un atome d'hydrogène ; et
R⁴ représente un groupe alkyle en C₁-C₄ ou R³ et R⁴, avec l'atome d'azote adjacent, forment un groupe amino cycloaliphatique ayant 5 ou 6 atomes formant le cycle, ledit groupe amino cycloaliphatique étant non substitué ou ayant au moins un des substituants (a) définis dans la revendication 1.

6. Utilisation selon la revendication 1, où :
R¹ représente un atome d'hydrogène, de fluor, de chlore ou de brome ;
R² représente un groupe phényle ou un groupe phényle substitué ayant au moins un des substituants (b) définis ci-après ;
R³ et R⁴, avec l'atome d'azote adjacent, forment un groupe morpholino, 1-pipérazinyle, 4-méthyl-1-pipérazinyle ou pipéridino ;
substituants (b) :
groupes alkyle en C₁-C₄, groupes alcoxy en C₁-C₄, groupes hydroxy, atomes de fluor, atomes de chlore et atomes de brome.

7. Utilisation selon la revendication 1, où :
R¹ représente un atome d'hydrogène ou un atome de chlore ;
R² représente un groupe phényle ou un groupe phényle substitué ayant au moins un substituant halogène ; et
R³ et R⁴, avec l'atome d'azote adjacent, forment un groupe morpholino, 1-pipérazinyle, 4-méthyl-1-pipérazinyle ou pipéridino.

8. Utilisation selon la revendication 1, où ledit dérivé d'isoxazoline-3-one est la 2-(2-hydroxy-3-morpholinopropyl)-5-phényl-4-isoxazoline-3-one ou un de ses sels d'addition d'acides pharmacologiquement acceptables.

9. Utilisation selon la revendication 1, où ledit dérivé d'isoxazoline-3-one est la 2-(2-hydroxy-3-morpholinopropyl)-5-phényl-4-isoxazoline-3-one ou son chlorhydrate.

10. Utilisation selon la revendication 1, où ledit dérivé d'isoxazoline-3-one est la 4-chloro-2-(2-hydroxy-3-morpholinopropyl)-5-phényl-4-isoxazoline-3-one ou un de ses sels d'addition d'acides pharmacologiquement acceptables.

11. Utilisation selon la revendication 1, où ledit dérivé d'isoxaioline-3-one est la 4-chloro-2-(2-hydroxy-3-morpholinopropyl)-5-phényl-4-isoxazoline-3-one ou son chlorhydrate.

12. Utilisation selon la revendication 1, où ledit dérivé d'isoxazoline-3-one est la 5-(p-chlorophényl)-2-(2-hydroxy-3-morpholinopropyl)-4-isoxazoline-3-one ou un de ses sels d'addition d'acides pharmacologiquement acceptables.

13. Utilisation selon la revendication 1, où ledit dérivé d'isoxazoline-3-one est la 5-(p-chlorophényl)-2-(2-hydroxy-3-morpholinopropyl)-4-isoxazoline-3-one ou son chlorhydrate.

14. Utilisation selon l'une quelconque des revendications précédentes, où ledit composé actif est formulé pour l'administration par voie orale ou par voie parentérale.

15. Utilisation selon l'une quelconque des revendications précédentes, où ledit composé actif est formulé sous forme de comprimés, de capsules, de granules, de poudres, de sirops, d'injections ou de suppositoires.

16. Utilisation selon l'une quelconque des revendications précédentes, où ledit composé actif est formulé pour l'administration à une dose de 5 mg à 50 mg par voie orale à des adultes humains, 1 à 3 fois par jour.
